(19) Europäisches Patentamt
European Patent Office
Office européen des brevets

(11) Veröffentlichungsnummer: **0 291 849 B1**

(12) **EUROPÄISCHE PATENTSCHRIFT**

(45) Veröffentlichungstag der Patentschrift: **16.10.91**

(51) Int. Cl.$^5$: **C07C 47/228**, C07C 45/50, C07C 45/75, A61K 7/46

(21) Anmeldenummer: **88107589.9**

(22) Anmeldetag: **11.05.88**

# (54) 4-Methyl-4-phenyl-1-pentanale, deren Herstellung und Verwendung als Riechstoffe.

(30) Priorität: **19.05.87 DE 3716730**

(43) Veröffentlichungstag der Anmeldung: **23.11.88 Patentblatt 88/47**

(45) Bekanntmachung des Hinweises auf die Patenterteilung: **16.10.91 Patentblatt 91/42**

(84) Benannte Vertragsstaaten: **CH DE ES FR GB LI NL**

(56) Entgegenhaltungen:
**EP-A- 0 219 092**
**DE-A- 2 101 489**
**DE-B- 2 855 505**
**FR-A- 2 499 069**

**JOURNAL OF ORGANIC CHEMISTRY, Band 29, Nr. 7, Seiten 1663-1669, 13. Juli 1964, American Chemical Society, W.M. URRY: "Free-Radical Rearrangements. II Ketones and Esters from the Reactions of aldehydes with Peroxides"**

(73) Patentinhaber: **BASF Aktiengesellschaft**
**Carl-Bosch-Strasse 38**
**W-6700 Ludwigshafen(DE)**

(72) Erfinder: **Gramlich, Walter, Dr.**
**Auf der Hoehe 11**
**W-6803 Edingen-Neckarhausen(DE)**
Erfinder: **Goetz, Norbert, Dr.**
**Schoefferstrasse 25**
**W-6520 Worms 1(DE)**
Erfinder: **Siegel, Hardo, Dr.**
**Hans-Purrmann-Allee 25**
**W-6720 Speyer(DE)**
Erfinder: **Schindler, Gerhard**
**Collinistrasse 5**
**W-6800 Mannheim 1(DE)**

**EP 0 291 849 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die Erfindung betrifft 4-Methyl-4-phenyl-1-pentanale der allgemeinen Formel I

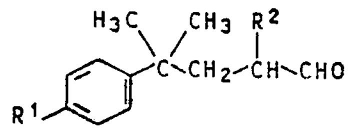

(I).

in der $R^1$ für Wasserstoff oder einen $C_1$- bis $C_4$-Alkylrest und $R^2$ für Wasserstoff oder eine Methylgruppe stehen, ausgenommen 4-Methyl-4-phenyl-1-pentanal, sowie die Herstellung und die Verwendung von 4-Methyl-4-phenyl-1-pentanalen der allgemeinen Formel I zur Verleihung, Verbesserung oder Modifizierung von Parfüms und parfümierten Produkten sowie Parfümzusammensetzungen.

Die 4-Methyl-4-phenyl-1-pentanale der allgemeinen Formel I sind neue Verbindungen, die besonders wertvolle Dufteigenschaften aufweisen. Nicht mehr neu ist lediglich 4-Methyl-4-phenyl-1-pentanal.

Diese Verbindung wurde von W.H. Urry u.a. in J. Org. Chem., Vol. 29 (1964) S. 1663 - 1669 in einer Arbeit mit dem Titel: "Free-Radical Rearrangements, II Ketones and Esters from the Reactions of Aldehydes Witz Peroxides" bereits beschrieben; in der Arbeit wird jedoch keinerlei Hinweise auf die interessanten olfaktorischen Eigenschaften dieser Verbindung gegeben.

Es wurde nun überraschenderweise gefunden, daß 4-Methyl-4-phenyl-1-pentanal einen Riechstoff darstellt, der einen hochinteressanten krautigen frischen Duft mit einer Holznote besitzt und interessante Effekte in Kompositionen aufweist.

Auch die neuen 4-Methyl-4-phenyl-1-pantanale sind farblose Flüssigkeiten, die hochinteressante Duftnoten aufweisen.

Es ist bereits eine Anzahl von Verbindungen mit einer gewissen strukturellen Beziehung zu den erfindungsgemäßen Verbindungen bekannt. Genannt seien beispielsweise:

A. 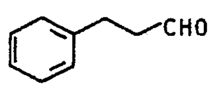 3-Phenyl-propanal (Dihydrozimtaldehyd)

B. 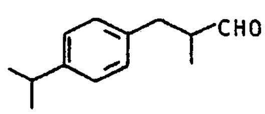 2-Methyl-3-(4-isopropyl-phenyl)-propanal (Cyclamenaldehyd)

C. 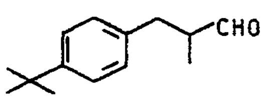 2-Methyl-3(4-tert.-butyl-phenyl)-propanal (Lilial®/Givaudan)

D. 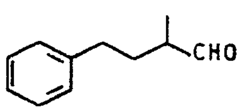 2-Methyl-4-phenyl-butanal

und

E. 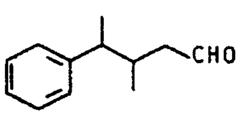 3-Methyl-4-phenyl-valeraldehyd

Die unter A genannte Verbindung besitzt einen stark blumigen Geruch mit einer schwach balsamischen und einer starken Hyacinthen-Note.

Die unter B und C genannten Verbindungen weisen beide einen intensiven Maiglöckchen-Duft auf.

Die unter D und E genannten Verbindungen besitzen nach Arctander (Parfume and Flavor Chemicals, Montclair 1969, Monographie Seiten 2172 und 2204) einen blumigen, grün-fruchtigen Geruch.

Die Verbindungen der allgemeinen Formel I weisen auch sehr wertvolle Dufteigenschaften auf, die sich aber von den Duftnoten der bekannten, strukturell verwandten und bekannten Verbindungen ganz wesentlich unterscheiden.

Gemäß J. Org. Chem. 29, S. 1663-69 wird 4-Methyl-4-phenyl-1-pentanal (Ia) auf dem folgenden Wege

hergestellt:

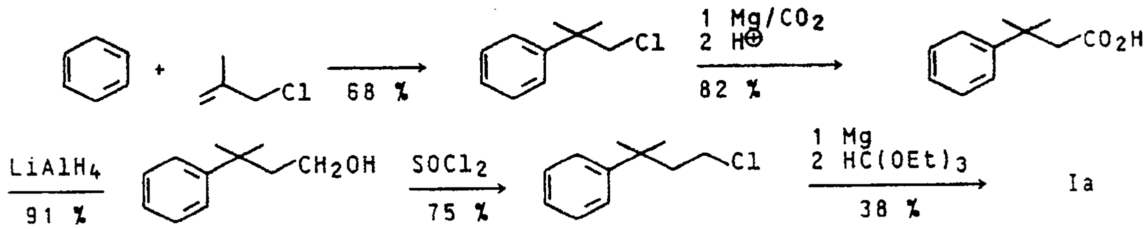


Das nach F.C. Withmore et al, J. Am. Chem. Soc. 65, (1943) 1469 durch Umsetzung von Benzol mit Methallylchlorid herstellbare 1-Chlor-2-methyl-2-phenyl-propan wird durch Grignard-Reaktion mit anschließender Trockeneis-Aufarbeitung und Hydrolyse in 82 % Ausbeute in die 3-Methyl-3-phenyl-buttersäure überführt, diese im nächsten Schritt mit Lithiumalanat zum Alkohol reduziert (91 % Ausbeute), dieser in einem weiteren Schritt mit Thionylchlorid in 75 proz. Ausbeute chloriert und das entstandene 1-Chlor-3-methyl-3-phenyl-propan mit Magnesium und danach mit Orthoameisensäureethylester zum 4-Methyl-4-phenyl-1-pentanal in nur 38 proz. Ausbeute überführt. Die bei diesem Verfahren erzielten Gesamtausbeuten betragen nur etwa 21 %. Nachteilig ist außerdem, daß die angewandten Reaktionschritte z.T. recht aufwendig sind. Dem gegenüber lassen sich Ia und darüber hinaus auch andere neue 4-Methyl-4-phenyl-1-pentanalen der allgemeinen Formel I mit überraschend guten olfaktorischen Eigenschaften auf einfache Weise aus den neuerdings recht gut zugänglichen 3-Methyl-3-phenyl-1-butenen der allgemeinen Formel II (vgl. die nicht vorveröffentliche Patentanmeldung P 35 36 929.9) in guten Ausbeuten herstellen.

Gegenstand der Erfindung ist daher auch ein Verfahren zur Herstellung von 4-Methyl-4-phenyl-1-pentanalen der allgemeinen Formel I das dadurch gekennzeichnet ist, daß man

A. die entsprechenden 3-Methyl-3-phenyl-1-butene der allgemeinen Formel II

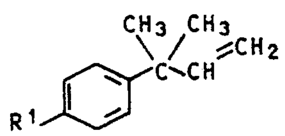
(II),

in der $R^1$ die in Anspruch 1 angegebene Bedeutung hat in an sich bekannter Weise hydroformyliert,

B. gegebenenfalls die dabei erhaltenen Methylphenylpentanale der allgemeinen Formel I, in der $R^2$ für Wasserstoff steht, mit Formaldehyd zu ungesättigten Verbindungen der allgemeinen Formel III

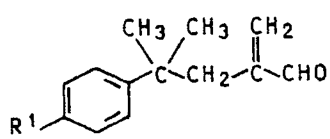
(III)

umsetzt und

C. diese dann selektiv zu den entsprechenden α-Methyl-phenylpentanalen der allgemeinen Formel I, in der $R^2$ = Methyl ist, hydriert.

Die Reaktionsbedingungen des Hydroformylierungsschrittes können in weiten Grenzen variiert werden.

Die Reaktionstemperatur liegt im allgemeinen zwischen 60 und 180 °C, bevorzugt zwischen 80 bis 110 °C.

Der Wasserstoff/Kohlenmonoxid-Druck beträgt allgemein zwischen 20 und 650 bar.

Als Hydroformylierungskatalysatoren verwendet man in der Regel Rhodiumverbindungen. Man verwendet sie im allgemeinen in Mengen von 0,1 bis 0,5 Gew.% Rhodium bezogen auf die Butene der Formel II. Besonders bewährt haben sich Mengen von 5 bis 200 ppm. Man kann Rhodiumcarbonylkomplexe für die Reaktion einsetzen oder andere Rhodiumverbindungen, die unter den Reaktionsbedingungen, Rhodiumcarbonylverbindungen bilden, wie beispielsweise die Halogenide, Oxide, Chelate oder fettsauren Salze des

Rhodiums.

Besonders vorteilhaft sind Rhodium-Komplexe oder Salze, die sich im Reaktionsgemisch homogen lösen, wie dimeres Rhodiumcarbonylchlorid, dimeres Cycloocta-1,5-dien-1-yl-rhodiumchlorid, Rhodiumcarbonylacetylacetonat oder Rhodium-2-ethyl-hexanoat.

Besonders vorteilhaft gelingt die Umsetzung, wenn man sie zusätzlich in Gegenwart von Triarylphosphinen oder Phosphiten, wie Triphenylphosphin oder Triphenylphosphit, durchführt.

Die Triarylphosphine der Phosphite verwendet man in einem bis zu 100-fach molaren Überschuß, bezogen auf Rhodium.

Die Hydroformylierungsprodukte werden nach Abtrennung des Katalysators in an sich bekannter Weise, z.B. durch Destillation aus dem Reaktionsgemisch isoliert.

Bei diesem Hydroformylierungsschritt werden 4-Methyl-4-phenyl-1-pentanale der allgemeinen Formel I, in der $R^2$ für Wasserstoff steht, erhalten.

Diese Verbindungen können dann gewünschtenfalls mit Formaldehyd in Form seiner wäßrigen Lösung oder als Paraformaldehyd in Gegenwart eines Katalysators zu ungesättigten Verbindungen der Formel III umgesetzt werden.

Als Katalysator eignen sich hierfür die in der Literatur beschriebenen Amine oder Enamine aus dem umzusetzenden Aldehyd mit z.B. Piperidin, Pyrrolidon oder Morpholin oder aber Gemische aus Aminen mit schwachen Säuren, wie Oxalsäure. Diese Reaktion wird bevorzugt bei Normaldruck zwischen 90 und 110 °C durchgeführt.

Für die nachfolgende selektive Hydrierung zum gesättigten Aldehyd der allgemeinen Formel I, in der $R^2$ für Methyl steht, verwendet man bevorzugt Edelmetall-Katalysatoren wie Palladium. Man setzt diese meist auf Trägern wie Aluminiumoxid, Titandioxid oder Aktivkohle ein. Die Katalysatoren enthalten im allgemeinen etwa 0,1 bis 5 Gew.%, vorzugsweise 0,5 bis 1 Gew.% Edelmetall, bezogen auf das Trägermaterial.

Die Hydrierbedingungen hängen wesentlich von der Menge und dem Inhalt des eingesetzten Katalysators ab.

Bei Verwendung eines 0,5 Gew.% Palladium enthaltenden Hydrierkatalysators, von dem 10 Gew.%, bezogen auf den ungesättigten Aldehyd, eingesetzt werden, verläuft die Reaktion im allgemeinen zwischen 80 und 140 °C und bei Wasserstoffdrücken zwischen 5 bis 50 bar.

Die Hydrierung läßt sich sowohl mit als auch ohne Lösungsmittel durchführen. Als Lösungsmittel können gegebenenfalls Kohlenwasserstoffe wie Cyclohexan, Alkohole, wie Methanol, oder Ester wie Essigsäureethylester eingesetzt werden.

Die Reinigung der nach Abtrennung vom Katalysator erhaltenen Rohprodukte erfolgt zweckmäßigerweise durch fraktionierte Destillation.

Die 4-Methyl-4-phenyl-1-pentanale der allgemeinen Formel I sind farblose Flüssigkeiten, die hochinteressante Geruchsnoten aufweisen.

Das 4-Methyl-4-phenyl-1-pentanal bringt aufgrund seines interessanten Geruches bereits bei geringer Dosierung in Kompositionen vom Fougère- und vom Chypre-Typ Ausdruck und Strahlkraft ein.

Es besitzt eine gute Haftfestigkeit und läßt sich gut mit üblichen Parfüminhaltsstoffen und anderen Riechstoffen zu neuartigen Kompositionen kombinieren.

Die bedeutendste der neuen Verbindung der Formel I ist das 2,4-Dimethyl-4-phenyl-pentanal. Es besitzt einen interessanten grünen Duft von frischen Blättern mit einer zusätzlichen holzigen Note.

Die erfindungsgemäßen Verbindungen der Formel I lassen sich sehr gut mit den üblichen Parfüminhaltsstoffen und anderen Riechstoffen zu neuartigen Kompositionen kombinieren, denen sie eine besondere Frische verleihen.

Der Anteil der 4-Methyl-4-phenyl-1-pentanale in den Riechstoffkompositionen beträgt im allgemeinen 1 bis 50 Gew.%.

Die auf diese Weise hergestellten Kompositionen können entweder direkt in der Fein-Parfümerie eingesetzt werden, oder aber zur Parfümierung von kosmetischen Präparaten, wie Cremes, Lotionen, Duftwässern, Toilettenseifen, Mundpflegemitteln, Aerosolen sowie in der Extrait-Parfümerie. Sie können weiterhin zur Geruchsverbesserung technischer Produkte wie Reinigungs- und Waschmitteln, Desinfektionsmitteln sowie Weichspülern dienen. Normalerweise werden 0,05 bis 2 Gew.% einer derartigen Komposition, bezogen auf das gesamte Produkt, eingesetzt.

Die erfindungsgemäßen Riechstoffe können einzeln oder aber insbesondere im Gemisch mit anderen Riechstoffen verwendet werden. Die Erfindung wird anhand von Beispielen erläutert.

Beispiel 1

A. Herstellung von 4-Methyl-4-phenyl-1-pentanal (Ia)

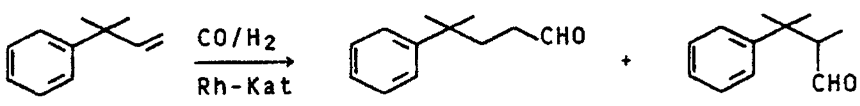


(IIa) (Ia) (IIIa)

In einem 1 l-Hochdruckautoklaven mit Magnetrührer wurden 710 g 3-Methyl-3-phenyl-1-buten, 42,7 mg $[Rh\text{-}Cyclooctadienyl\text{-}1{,}5\text{-}Cl]_2$ (100 ppm), sowie 1,42 g Triphenylphosphin (1000 ppm) vorgelegt. Man preßte bei Raumtemperatur 100 bar $CO/H_2$ (1:1) auf und erwärmte dann auf 100° C. Danach wurde der Druck auf 300 bar $CO/H_2$ (1:1) erhöht. Man ließ Druck und Temperatur während 12 Stunden (h) konstant, kühlte ab und entspannte.

Eine gaschromatographische Analyse (GC-Analyse) zeigte einen Umsatz von 94 %. Das Verhältnis von 4-Methyl-4-phenyl-1-pentanal (Ia) zu 2,3-Dimethyl-3-phenyl-1-butanal (IIIa) lag bei 93:7. Ia ließ sich leicht destillativ von IIIa abtrennen.

Man erhielt reines 4-Methyl-4-phenyl-pentanal in einer Ausbeute von 82%, bezogen auf IIa.

Kp = 70° C/0.01 mbar, $n_D^{25}$ 1.5131

Duftnote: krautig frisch mit einer Holznote.

B. Anwendung von Ia zur Modifizierung einer Parfüm-Komposition von Fougère-Typ (A).

| | A | B |
|---|---|---|
| Anisaldehyd (BASF) | 30 | 30 |
| Linalool (BASF) | 150 | 150 |
| Lavandinöl abrialis | 50 | 50 |
| Lavendelöl Mont Blanc | 70 | 70 |
| α-Hexyl-zimtaldehyd | 50 | 50 |
| Lilial* (Givaudan) | 50 | 50 |
| Phenylethanol st. (BASF) | 50 | 50 |
| Amylsalicylat | 20 | 20 |
| Cyclohexylethylacetat (BASF) | 30 | 30 |
| Rosmarinöl tunesisch | 30 | 30 |
| Sandelether* (BASF) | 40 | 40 |
| Musk Ether* (FDO) | 40 | 40 |
| Geraniol | 30 | 30 |
| Citronellol (BASF) | 30 | 30 |
| Coumarin | 20 | 20 |
| Vetiveröl Haiti | 20 | 20 |
| Patchouliöl Singapore | 20 | 20 |
| Labdanumextrakt | 20 | 20 |
| Styrolylacetat | 20 | 20 |
| Geraniumöl Bourbon | 20 | 20 |
| Eucalyptusöl glob. | 10 | 10 |
| Eichenmoosextrakt | 10 | 10 |
| Lavandin* absolute | 10 | 10 |
| Opoponax Extrakt | 20 | 20 |
| Eugenol | 10 | 10 |
| Hedione* | 10 | 10 |
| Hydroxycitronellal (BASF) | 50 | 50 |
| Dipropylenglykol | 90 | 0 |
| 4-Methyl-4-phenyl-1-pentanal | 0 | 90 |
| | 1000 | 1000 |

* = angemeldetes Warenzeichen

Die bekannte, oben in seiner Zusammensetzung beschriebene Parfümkomposition A besitzt einen frisch-krautigen Charakter. Ersetzt man 90 Teile Dipropylenglykol durch das erfindungsgemäße 4-Methyl-4-phenyl-1-pentanal (Komposition B), so bewirkt dies eine leicht abrundende modifizierende Wirkung in der grünen Fondnote der Komposition. Die Ia enthaltende Komposition besitzt überdies eine bessere Verbreitung und eine zunehmende Fülle.

Die Komposition eignet sich in besonderem Maße für Badepräparate.

Beispiel 2

4-Methyl-4-(p-methyl-phenyl)-1-pentanal (Ib)

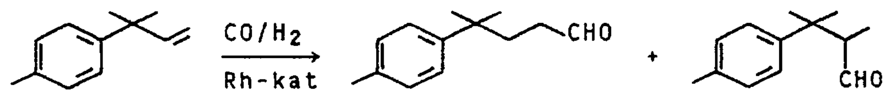


(IIb) (Ib) (IIIb)

in einem 1 l-Hochdruckautoklaven mit Magnetrührer wurden 710 g (4,4 mol) 3-Methyl-3-(p-methyl-phenyl)-1-buten, 42,7 mg [Rh-Cyclooctadienyl-1,5-Cl]$_2$ (100 ppm), sowie 1,42 g Triphenylphosphin (1000 ppm) vorgelegt. Man preßte bei Raumtemperatur 100 bar $CO/H_2$ (1:1) auf und erwärmte dann auf 100 °C. Danach wurde der Druck auf 300 bar $CO/H_2$ (1:1) erhöht. Man ließ Druck und Temperatur während 12 h konstant, kühlte ab und entspannte.

Eine GC-Analyse zeigte einen Umsatz von 94 %. Das Verhältnis von 4-Methyl-4-(p-methyl-phenyl)-1-pentanal (Ib) zu 2,3-Dimethyl-3(p-methyl-phenyl)-1-butanal (IIIb) lag bei 93:7. Ib ließ sich leicht destillativ von IIIb abtrennen.

Man erhielt reines 4-Methyl-4-(p-methyl-phenyl)-1-pentanal in einer Ausbeute von 82 % der Theorie, bezogen auf IIb.
Kp 114 °C/1 mbar; $n_D^{25}$ 1.5138

| | |
|---|---|
| IR (Film): | 2964, 2925, 2873, 1724, 1516, 1456, 1388, 1366, 818, 588 $cm^{-1}$ |
| $^1$H-NMR ($CDCl_3$): | $\delta$ = 9,58 (s, 1H), 7.4-7.1 (m, 4H), 2.31 (s, 3H), 2,2 (t, 2H), 1.92 (t, 2H), 1.02 (s, 6H) ppm. |
| MS (m/e): | $M^+$ = 190 (16 %), 157 (2 %), 142 (1.5), 133 (100), 115 (4), 105 (21), 93 (6), 77 (4), 65 (3), 51 (1), 41 (9). |

Duftnote: interessante würzige Note.

Beispiel 3

Man arbeitete analog Beispiel 1, verwendete jedoch anstelle von 710 g IIb 889 g 3-Methyl-3-(p-tert.-butyl-phenyl)-1-buten für die Hydroformylierungsreaktion.

Man erhielt 4-Methyl-4-(p-tert.-butyl-phenyl)-1-pentanal vom Kp 135 °C/1,0 mbar in einer Ausbeute von 81 % der Theorie.

$n_D^{25}$ 1.5080

| | |
|---|---|
| IR (Film) | 2963, 2900, 2870, 1725, 1512, 1402, 1363, 1271, 832, 583 $cm^{-1}$. |
| $^1$H-NMR ($CDCl_3$) $\delta$ | = 9.5 (s, 1H), 7.2-7.0 (m, 4H), 2.2 (t, 2H), 1.94 (t, 2H), 1.31 (s, 6H), 3.0 (s, 9H) ppm. |
| MS (m/e): | $M^+$ = 232 (9 %), 175 (100), 160 (10), 145 (8), 131 (4), 117 (5), 105 (3), 91 (6), 81 (4), 69 (2), 57 (26), 41 (13). |

Duftnote: grün-kampfriger Duft, schwach krautige Note.

Beispiel 4

2,4-Dimethyl-4-phenyl-1-pentanal

Man erhitzte unter Rühren ein Gemisch aus 826 g (3 mol) 4-Methyl-4-phenyl-1-pentanal, 95 g Paraformaldehyd, 500 ml $H_2O$ und 42 g eines zuvor hergestellten Enamins aus 4-Methyl-4-phenyl-1-pentanal und Piperidin als Katalysator 2 h unter Rückfluß zum Sieden.

Anschließend kühlte man ab, trennte die wäßrige Phase ab, verdünnte die organische Phase mit Methanol (1:1), gab 80 g eines $Pd/Al_2O_3$-Katalysators hinzu und hydrierte in einem 3 l Autoklaven für
3 h bei 25 bar $H_2$, 4 h bei 35 bar $H_2$,
4 h bei 45 bar $H_2$ und 4 h bei 55 bar $H_2$.
Anschließend entspannte man den Autoklaven und arbeitete destillativ auf.

Man erhielt das gewünschte 2,4-Dimethyl-4-phenyl-1-pentanal in 77 %iger Ausbeute, bezogen auf eingesetztes 4-Methyl-4-phenyl-1-pentanal.
Kp 86 °C/0,3 mbar, $n_D^{25}$ 1.5070

| | |
|---|---|
| IR (Film): | 2965, 2933, 2875, 1724, 1497, 1456, 1447, 1368, 766 701 $cm^{-1}$. |

$^1$H-NMR ($CDCl_3$): $\delta$ = 0,9 (d, 3H), 1,31 (s, 3H), 1,33 (s, 3H), 1,5-1,6 (m, 1H), 2,1-2,35 (m, 2H), 7.1-7.5 (m, 5H), 9,26 (s, 1H) ppm.

MS (m/e): $M^+$ = 190 (4 %), 157 (2 %), 134 (8), 119 (100), 105 (7), 91 (36), 77 (5), 65 (2), 51 (2), 41 (16).

Duftnote: interessanter krautig-holziger Duft.

**Patentansprüche**

1. 4-Methyl-4-phenyl-1-pentanale der allgemeinen Formel I

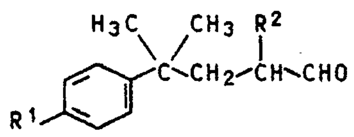

(I),

in der $R^1$ für Wasserstoff oder einen $C_1$- bis $C_4$-Alkylrest und $R^2$ für Wasserstoff oder eine Methylgruppe stehen, ausgenommen 4-Methyl-4-phenyl-1-pentanal.

2. Verwendung von 4-Methyl-4-phenyl-1-pentanalen der allgemeinen Formel I zur Verleihung, Verbesserung oder Modifizierung der Dufteigenschaften von Parfüms und parfümierten Produkten.

3. Riechstoffkompositionen, gekennnzeichnet durch einen Gehalt von 1 bis 50 Gew.% an 4-Methyl-4-phenyl-1-pentanalen der allgemeinen Formel I.

4. Verwendung von 4-Methyl-4-phenyl-1-pentanal zur Verleihung, Verbesserung oder Modifizierung der Dufteigenschaften von Parfüms und parfümierten Produkten.

5. Riechstoffkompositionen enthaltend 4-Methyl-4-phenyl-1-pentanal.

6. Verfahren zur Herstellung von 4-Methyl-4-phenyl-1-pentanalen der allgemeinen Formel I, dadurch gekennzeichnet, daß man

   A. die entsprechenden 3-Methyl-3-phenyl-1-butene der allgemeinen Formel II

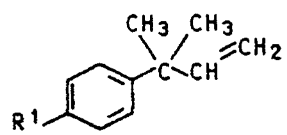

(II),

   in der $R^1$ die in Anspruch 1 angegebene Bedeutung hat in an sich bekannter Weise hydroformyliert,

   B. gegebenenfalls das dabei erhaltene Methylphenylpentanal der allgemeinen Formel I, in der $R^2$ für Wasserstoff steht, mit Formaldehyd zu ungesättigten Verbindungen der allgemeinen Formel III

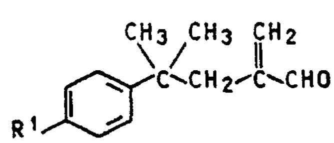

(III)

   unsetzt und

   C. diese dann selektiv zu dem entsprechenden $\alpha$-Methyl-phenylpentanal der allgemeinen Formel I, in der $R^2$ = Methyl ist, hydriert.

**7.** Verfahren zur Herstellung von 4-Methyl-4-phenyl-1-pentanalen der allgemeinen Formel I gemäß Anspruch 7, dadurch gekennzeichnet, daß man die Hydroformylierung im Reaktionsschritt A mit einer Rhodiumverbindung als Hydroformylierungskatalysator durchführt.

**8.** 2,4-Dimethyl-4-phenyl-1-pentanal.

**9.** 4-Methyl-4(p-methyl-phenyl)-1-pentanal.

**10.** 4-Methyl-4(p-tert.-butyl-phenyl)-1-pentanal.

## Claims

**1.** A 4-methyl-4-phenyl-1-pentanal of the formula I

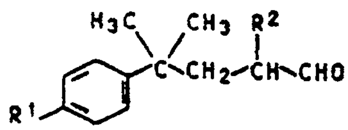

(I)

where $R^1$ is hydrogen or $C_1$-$C_4$-alkyl and $R^2$ is hydrogen or methyl, with the exception of 4-methyl-4-phenyl-1-pentanal.

**2.** Use of a 4-methyl-4-phenyl-1-pentanal of the formula I for imparting fragrance properties to, or improving or modifying the fragrance properties of, perfumes and perfumed products.

**3.** A scent composition containing from 1 to 50% by weight of a 4-methyl-4-phenyl-1-pentanal of the formula I.

**4.** Use of a 4-methyl-4-phenyl-1-pentanal for imparting fragrance properties to, or improving or modifying the fragrance properties of, perfumes and perfumed products.

**5.** A scent composition containing 4-methyl-4-phenyl-1-pentanal.

**6.** A process for the preparation of a 4-methyl-4-phenyl-1-pentanal of the formula I, wherein

A. the corresponding 3-methyl-3-phenyl-1-butene of the formula II

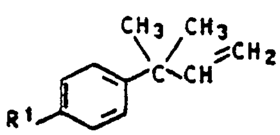

(II)

where $R^1$ has the meanings stated in claim 1, is hydroformylated in a conventional manner,

B. the resulting methylphenylpentanal of the formula I, where $R^2$ is hydrogen, is, if required, reacted with formaldehyde to give an unsaturated compound of the formula III

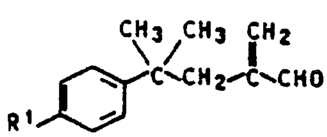

(III)

and

C. this is then selectively hydrogenated to give the corresponding $\alpha$-methylphenylpentanal of the

formula I, where $R^2$ is methyl.

7. A process for the preparation of a 4-methyl-4-phenyl-1-pentanal of the formula I as claimed in claim 7, wherein the hydroformylation in reaction step A is carried out using a rhodium compound as the hydroformylation catalyst.

8. 2,4-dimethyl-4-phenyl-1-pentanal.

9. 4-methyl-4-(p-methylphenyl)-1-pentanal.

10. 4-methyl-4-(p-tert-butylphenyl)-1-pentanal.

**Revendications**

1. 4-Méthyl-4-phényl-1-pentanals de formule générale

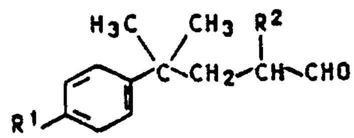

(I),

dans laquelle $R^1$ est mis pour un atome d'hydrogène ou un reste alkyle en $C_1$ à $C_4$ et $R^2$ pour un atome d'hydrogène ou un groupement méthyle, à l'exception du 4-méthyl-4-phényl-1-pentanal.

2. Utilisation de 4-méthyl-4-phényl-1-pentanals de formule générale I pour donner des propriétés odoriférantes à des parfums et à des produits parfumés ou pour améliorer ou modifier ces propriétés.

3. Compositions de matières odorantes, caractérisées par une teneur de 1 à 50% en poids de 4-méthyl-4-phényl-1-pentanals de formule générale I.

4. Utilisation de 4-méthyl-4-phényl-1-pentanal pour donner, améliorer ou modifier des propriétés odoriférantes à des parfums et à des produits parfumés ou pour améliorer ou modifier ces propriétés.

5. Compositions de matières odorantes contenant du 4-méthyl-4-phényl-1-pentanal.

6. Procédé de préparation de 4-méthyl-4-phényl-1-pentanals de formule générale I, caractérisé en ce que
A. on hydroformyle, de façon connue en soi les 3-méthyl-3-phényl-1-butènes correspondants de formule générale II

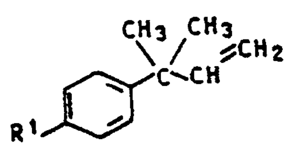

(II),

dans laquelle $R^1$ a la signification donnée dans la revendication 1;
B. on fait éventuellement réagir le méthylphénylpentanal obtenu, de formule générale I dans laquelle $R^2$ est mis pour un atome d'hydrogène, avec du formaldéhyde pour obtenir des composés insaturés de formule générale III

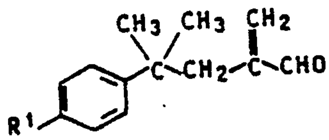

(III)

puis

C. on hydrogène sélectivement ceux-ci pour obtenir l'∝-méthylphénylpentanal correspondant de formule générale I, dans laquelle $R^2$ = méthyle.

**7.** Procédé de préparation de 4-méthyl-4-phényl-1-pentanals de formule générale I selon la revendication 6, caractérisé en ce qu'on conduit l'hydroformylation, dans l'étape réactionnelle A, avec un composé du rhodium comme catalyseur d'hydroformylation.

**8.** 2,4-Diméthyl-4-phényl-1-pentanal.

**9.** 4-Méthyl-4(p-méthylphényl)-1-pentanal.

**10.** 4-Méthyl-4(p-tert.-butylphényl)-1-pentanal.